(19) **Europäisches Patentamt / European Patent Office / Office européen des brevets**

(11) **EP 4 424 290 B1**

(12) **EUROPEAN PATENT SPECIFICATION**

(45) Date of publication and mention
of the grant of the patent:
**22.04.2026   Bulletin 2026/17**

(51) International Patent Classification (IPC):
**A61L 15/18** (2006.01)    **A61L 15/24** (2006.01)
**A61L 15/60** (2006.01)    **A61F 13/49** (2006.01)
**A61F 13/53** (2006.01)

(21) Application number: **23159812.9**

(22) Date of filing: **03.03.2023**

(52) Cooperative Patent Classification (CPC):
(C-Sets available)
**A61F 13/49; A61L 15/18; A61L 15/24; A61L 15/60;**
A61F 2013/530481; A61F 2013/530671      (Cont.)

(54) **METHOD FOR PREPARING AN ABSORBENT ARTICLE COMPRISING SUPERABSORBENT POLYMER, USING A COMPOSITION COMPRISING A CLAY DISPERSION**

VERFAHREN ZUR HERSTELLUNG EINES ABSORBIERENDEN ARTIKELS, DER EIN SUPERABSORBIERENDES POLYMER ENTHÄLT, UNTER VERWENDUNG EINER ZUSAMMENSETZUNG, DIE EINE TONDISPERSION ENTHÄLT

PROCÉDÉ DE PRÉPARATION D'UN ARTICLE ABSORBANT COMPRENANT UN POLYMÈRE SUPERABSORBANT, UTILISANT UNE COMPOSITION COMPRENANT UNE DISPERSION D'ARGILE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**04.09.2024   Bulletin 2024/36**

(73) Proprietor: **The Procter & Gamble Company
Cincinnati, OH 45202 (US)**

(72) Inventors:
• **SIMONYAN, Arsen
  65824 Schwalbach am Taunus (DE)**
• **BORGMANN, Carolin
  65824 Schwalbach am Taunus (DE)**
• **WON, TaeYoung
  17336 Seoul (KR)**
• **SOHN, Jung-min
  17336 Seoul (KR)**
• **JANG, Youngjin
  Seoul 17336 (KR)**
• **YOON, Ki Youl
  Seoul 17336 (KR)**
• **HAN, Chang Hun
  Seoul 17336 (KR)**

(74) Representative: **P&G Patent Germany
Procter & Gamble Service GmbH
Sulzbacher Straße 40
65824 Schwalbach am Taunus (DE)**

(56) References cited:
**EP-A1- 2 277 558      EP-A1- 3 391 960
WO-A1-2009/041870    US-A1- 2010 100 066**

**(Cont. next page)**

(52) Cooperative Patent Classification (CPC): (Cont.)

C-Sets
**A61L 15/24, C08L 39/06**

## Description

Technical Field

The present invention relates to a clay dispersion, for application in making superabsorbent polymer to be incorporated in absorbent articles, the dispersion having excellent dispersion stability, a composition for preparing a superabsorbent polymer for use in absorbent articles, the composition including the clay dispersion, and a method of preparing a superabsorbent polymer using the clay dispersion, wherein the superabsorbent polymer is incorporated into an absorbent article.

Background of the Invention

A superabsorbent polymer (SAP) is a synthetic polymeric material capable of absorbing moisture from 500 to 1000 times its own weight. Since superabsorbent polymers started to be practically applied in absorbent articles, now they have been widely used not only for hygiene products such as disposable diapers for children, sanitary materials for sanitary napkins, etc., but also for water retaining soil products for gardening, water stop materials for the civil engineering and construction, sheets for raising seedling, fresh-keeping agents for food distribution fields, materials for poultice, or the like.

In the superabsorbent polymers, a water-soluble ethylenically unsaturated monomer is used, and one or more monomers selected from the group consisting of an anionic monomer and a salt thereof, a nonionic hydrophilic monomer, an amino group-containing unsaturated monomer and a quaternary thereof may be used. The water-soluble ethylenically unsaturated monomer is neutralized with an alkali metal salt such as a sodium salt, etc., or a basic compound such as caustic soda, and then may be polymerized by including a crosslinking agent and a polymerization initiator, and this solution is referred to as a neutralization solution or a composition for preparing a superabsorbent polymer. This neutralization solution is subjected to thermal polymerization or photopolymerization to prepare a water-containing gel polymer, which is then dried, pulverized, and classified to prepare a powdered superabsorbent polymer.

Studies have been conducted on introducing a clay as an additive in order to improve basic physical properties such as centrifuge retention capacity (CRC) and absorbency under pressure (AUP) of the superabsorbent polymer. However, when the clay is introduced into the composition for preparing the superabsorbent polymer, there is a problem that it is not easy to secure dispersion stability because the clay particles tend to agglomerate with each other due to high ion concentrations of the alkali metal salt or the basic compound which is added for neutralization. The clay thus agglomerated is non-evenly distributed in the form of particles in the polymer, and consequently, the effect of improving physical properties of the polymer may not be obtained.

Accordingly, it is required to develop a clay dispersion capable of exhibiting excellent dispersibility in the composition for preparing the superabsorbent polymer and improving physical properties of the superabsorbent polymer.

EP3391960 relates to superabsorbent polymer particles which comprise clay platelets with edge modification and/or surface modification.

WO2009041870 discloses a charged claylinked gel. The charged claylinked gel comprises clay nanoparticles which are crosslinked by a charged polymer.

Technical Problem

The following describes a clay dispersion for application in making superabsorbent polymer to be incorporated in absorbent articles, the clay dispersion exhibiting excellent dispersion stability.

The following also describes a composition for preparing a superabsorbent polymer for use in absorbent articles, the composition including the clay dispersion.

There is also provided a method of preparing a superabsorbent polymer using the clay dispersion, wherein the superabsorbent polymer is subsequently incorporated in an absorbent article.

Technical Solution

The following describes a clay dispersion for application in making superabsorbent polymer, the superabsorbent polymer being subsequently incorporated in absorbent articles, the clay dispersion including a solvent; a clay; and a polymer dispersant including two or more functional groups selected from the group consisting of an amine group, a carbonyl group, and a hydroxyl group, wherein the polymer dispersant is included in an amount of more than 20 parts by weight to 200 parts by weight or less with respect to 100 parts by weight of the clay.

The following also describes a composition for preparing a superabsorbent polymer for use in absorbent articles, the composition including the clay dispersion; a water-soluble ethylenically unsaturated monomer; an alkali metal salt or alkali compound capable of neutralizing the water-soluble ethylenically unsaturated monomer; and a polymerization initiator.

The absorbent article comprising the superabsorbent polymer made by using the clay dispersion and/or by using the composition, may be a diaper or a pant.

The superabsorbent polymer may be incorporated into an absorbent core, the absorbent core being comprised by the absorbent article.

The absorbent core may be made by providing two nonwoven webs and incorporating the surface-crosslinked particulate water-absorbing resin between the two nonwoven webs.

The absorbent core, in between the two nonwoven webs, may comprise less than 20 weight-% of cellulose fibers, preferably less than 10 weight-% of cellulose fibers, and more preferably less than 5 weight-% of cellulose fibers.

In the absorbent core, the superabsorbent polymer may be adhesively immobilized in between the two nonwoven webs.

The absorbent article may comprise a topsheet, a backsheet and the absorbent core in between the topsheet and the backsheet.

According to the present invention, there is provided a method of making an absorbent article comprising superabsorbent polymer, wherein the superabsorbent polymer is prepared by using a composition, the composition comprising a clay dispersion; a water-soluble ethylenically unsaturated monomer; an alkali metal salt or alkali compound capable of neutralizing the water-soluble ethylenically unsaturated monomer; and a polymerization initiator; the clay dispersion comprising:

> a solvent;
> a clay; and
> a polymer dispersant including two or more functional groups selected from the group consisting of an amine group, a carbonyl group, and a hydroxyl group,
> wherein the polymer dispersant is included in an amount of more than 20 parts by weight to not more than 200 parts by weight with respect to 100 parts by weight of the clay;
> wherein, in the composition, the content of the clay is from 0.1 part by weight to 1 part by weight with respect to 100 parts by weight of the ethylenically unsaturated monomer; and
> wherein, in the composition, the clay has a sedimentation rate of 100 $\mu$m/s or less.

Effect of the Invention

A clay dispersion used in the method of the present invention has a low sedimentation rate of clay particles and excellent dispersion stability even during long-term storage, particularly, it exhibits excellent dispersion stability for a long period of time even in a composition for preparing a superabsorbent polymer, the composition including an alkali metal salt or a basic material. Therefore, the clay dispersion may be usefully used as an additive for improving physical properties of the superabsorbent polymer, such as centrifuge retention capacity and absorbency under pressure, etc. In addition, there is no concern about discoloration of the superabsorbent polymer which is prepared using the clay dispersion.

Brief Description of Drawings

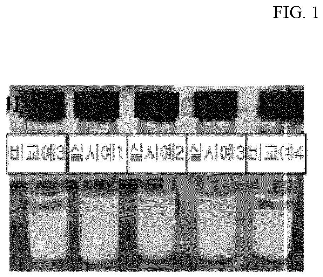

FIG. 1 is a photograph showing compositions for preparing a superabsorbent polymer of Examples 1-1 to 1-3, Comparative Example 1-3, and Comparative Example 1-4, which was taken 48 hours after preparation of the compositions.

Detailed description of the Invention

The language and terms used in this description are just for explaining exemplary embodiments and it is not intended to restrict the present invention. The singular expression may include the plural expression unless it is differently expressed contextually. It must be understood that the term "include", "equip", or "have" in the present description is only used for designating the existence of characteristics taken effect, steps, components, or combinations thereof, and do not exclude the existence or the possibility of addition of one or more different characteristics, steps, components, or combinations thereof beforehand.

As used herein, 'clay' refers to a particle of a phyllosilicate mineral, or an aggregate of plurality of such particles, and 'clay dispersion' refers to a dispersion in which the clay is dispersed in a solvent.

As used herein, (meth)acrylate is used as a meaning including both acrylate and methacrylate.

As used herein, "base polymer" or "base polymer powder", which is in a particle or powder form prepared by drying or pulverizing a polymer which is obtained by polymerizing a water-soluble ethylenically unsaturated monomer, refers to a polymer without surface modification or surface crosslinking.

Hereinafter, the present invention will be described in detail.

A clay dispersion is characterized by including a solvent; a clay; and a polymer dispersant including two or more functional groups selected from the group consisting of an amine group, a carbonyl group, and a hydroxyl group, wherein the polymer dispersant is included in an amount of more than 20 parts by weight to 70 parts by weight or less with respect to 100 parts by weight of the clay.

Due to the polymer dispersant including two or more functional groups selected from the group consisting of an amine group, a carbonyl group, and a hydroxyl group, the clay dispersion may remarkably improve dispersion stability of clay. Clay has a layered structure in which layers of small plates of silicate of about 1 nm thickness are stacked on top of each other due to strong van der Waals attraction. The polymer dispersant binds to the surface of the clay and delaminates the silicate layers (i.e., spreads them apart so that the interlayer distance exceeds 1 nm). As the chemically delaminated clay exhibits significantly improved dispersion stability, such that even when the clay dispersion included in a composition for preparing a superabsorbent polymer including an alkali metal salt or a basic compound, clay particles may be uniformly dispersed in the composition without agglomeration or sedimentation.

Therefore, a superabsorbent polymer prepared using the clay dispersion may have further improved absorption properties, such as centrifuge retention capacity, absorbency under pressure, etc. In addition, since the clay dispersion does not cause discoloration of the superabsorbent polymer, it may be suitably used for manufacturing high-quality products.

As the clay, swelling or non-swelling clay may be used. The swelling clay is a layered organic material having water absorption, and montmorillonite, saponite, nontronite, laponite, beidellite, hectorite, vermiculite, magadite, bentonite, etc. may be used. As the non-swellable clay, kaolin, serpentine, and mica, etc. may be used. The clay may be used alone or in mixture of two or more thereof.

The clay having an average particle diameter of 0.025 μm or more, or 0.05 μm or more, or 0.1 μm or more, or 1 μm, and 10 μm or less, or 8 μm or less, or 5 μm or less may be used. When the average particle diameter of the clay is less than 0.025 μm, the effect of improving gel strength of the superabsorbent polymer may not be sufficiently obtained. When the average particle diameter of the clay is more than 10 μm, it is difficult to achieve uniform dispersion in a water system due to the strong interlayer attraction of the clay, and it is difficult to uniformly modify the surface of the clay, and thus there may be a problem in that transparency is not secured after dispersion. The average particle diameter of the clay may be measured through a laser diffraction and dynamic light scattering method using a particle size analyzer.

The polymer dispersant includes two or more functional groups selected from the group consisting of an amine group, a carbonyl group, and a hydroxyl group, and is used in an amount of more than 20 parts by weight to 200 parts by weight or less with respect to 100 parts by weight of the clay.

As used herein, 'two or more functional groups selected from the group consisting of an amine group, a carbonyl group, and a hydroxyl group' is used as a meaning including two or more functional groups of an amine group, a carbonyl group, and a hydroxyl group in combination, such as an amide group including an amine group and a carbonyl group at the same time, a carboxyl group including a carbonyl group and a hydroxyl group at the same time, etc. For example, the 'polymer dispersant including an amine group and a carbonyl group' may include the amine group and the carbonyl group in the molecule, respectively, or may include an amide group in which the amine group and the carbonyl group are in combination.

The polymer dispersant including the functional groups may bind to the surface of the clay due to unshared electrons of nitrogen and oxygen atoms, and charges may be induced in the polymer due to a resonance phenomenon in the composition for preparing a superabsorbent polymer with high ion concentrations. Due to these properties, the polymer dispersant may improve dispersibility of clay by delaminating its layered structure. Moreover, the polymer dispersant does not cause discoloration of the superabsorbent polymer or does not deteriorate physical properties thereof, and therefore, may be suitably used in the preparation of the superabsorbent polymer.

In this point of view, the polymer dispersant may preferably include an amine group and a carbonyl group; or a carbonyl group and a hydroxyl group. More preferably, the polymer dispersant may include an amide group and/or a carboxyl group.

Specific examples of the polymer dispersant may include one or more selected from the group consisting of polyvinylpyrrolidone, polyacrylamide, and polyacrylic acid. Preferably, polyvinylpyrrolidone may be used as the polymer dispersant.

A molecular weight of the polymer dispersant is not particularly limited. However, for example, the polymer dispersant having a weight average molecular weight of 2,500 g/mol or more, or 5,000 g/mol or more, or 10,000 g/mol or more, and 200,000 g/mol or less, or 100,000 g /mol or less, or 40,000 g/mol may be used. When the weight average molecular weight of the polymer dispersant is less than 2,500 g/mol, there may be problems in securing dispersibility and long-term stability because it is difficult to secure a sufficient distance between the silicate layers of clay when the polymer dispersant binds to the surface of the clay. When the weight average molecular weight is more than 200,000 g/mol, there is a difficulty in the process, and it is not effective for surface modification of the clay, and thus it is preferable to satisfy the above range.

In order to ensure dispersibility of the clay, the polymer dispersant is used in an amount of more than 20 parts by weight to 200 parts by weight or less, preferably, in an amount of 23 parts by weight or more, or 25 parts by weight or more, and 150 parts by weight or less, or 100 parts by weight or less with respect to 100 parts by weight of the clay.

When the content of the polymer dispersant is 20 parts by weight or less with respect to 100 parts by weight of the clay, the degree of surface modification of the clay is insufficient and thus the interlayer spacing of the clay may not be sufficiently widened. Therefore, the dispersion stability of the clay is not sufficient, and thus the clay particles may easily agglomerate. In addition, when the content of the polymer dispersant is excessively high by exceeding 200 parts by weight with respect to 100 parts by weight of the clay, the polymer dispersant reduces the effect of improving physical properties, which may be provided by the clay, leading to deterioration of the physical properties of the superabsorbent polymer.

The kind of the solvent used in the clay dispersion is not particularly limited, and any solvent may be used without limitation as long as it is used in the composition for preparing the superabsorbent polymer. For example, one or more selected from the group consisting of water, ethanol, ethylene glycol, diethylene glycol, triethylene glycol, 1,4-butanediol, propylene glycol, ethylene glycol monobutyl ether, propylene glycol monomethyl ether, propylene glycol monomethyl ether acetate, methyl ethyl ketone, acetone, methyl amyl ketone, cyclohexanone, cyclopentanone, diethylene glycol monomethyl ether, diethylene glycol ethyl ether, toluene, xylene, butyrolactone, carbitol, methyl cellosolve acetate, and N,N-dimethylacetamide may be used as the solvent.

The amount of the solvent used for the preparation of the clay dispersion is not limited, and the amount of solvent may be appropriately adjusted in consideration of the kind and amount of the clay and the polymer dispersant, and the use of the clay dispersion.

The content of the clay in the clay dispersion may be 1% by weight or more, or 3% by weight or more, and 20% by weight or less, or 10% by weight or less, or 8% by weight or less. When the content of the clay is satisfied, clay particles may maintain excellent dispersibility without agglomeration with each other or precipitation even during long-term storage.

Meanwhile, provided is a composition for preparing a superabsorbent polymer, the composition including the clay dispersion; a water-soluble ethylenically unsaturated monomer; an alkali metal salt or alkali compound capable of neutralizing the water-soluble ethylenically unsaturated monomer; and a polymerization initiator.

Description of the clay dispersion is the same as described above.

The clay dispersion may be used such that the content of the clay is 0.1 part by weight or more, or 0.2 parts by weight or more, or 0.25 parts by weight or more, and 1 part by weight or less, or 0.7 parts by weight or less, or 0.5 parts by weight or less, based on 100 parts by weight of the water-soluble ethylenically unsaturated monomer included in the composition for preparing a superabsorbent polymer. **In** other words, the content of the clay in the composition for preparing a superabsorbent polymer may be 0.1 part by weight or more, or 0.2 parts by weight or more, or 0.25 parts by weight or more, and 1 part by weight or less, or 0.7 parts by weight or less, or 0.5 parts by weight or less, based on 100 parts by weight of the ethylenically unsaturated monomer.

As the water-soluble ethylenically unsaturated monomer, any monomer may be used without particular limitation, as long as it is commonly used in the preparation of superabsorbent polymers. Preferably, any one or more selected from the group consisting of anionic monomers and salts thereof, nonionic hydrophilic monomers, and amino group-containing unsaturated monomers and quaternary compounds thereof may be used. Specific examples of the water-soluble ethylenically unsaturated monomer may include one or more selected from the group consisting of anionic monomers such as acrylic acid, methacrylic acid, maleic anhydride, fumaric acid, crotonic acid, itaconic acid, 2-acryloyl ethane sulfonic acid, 2-methacryloyl ethane sulfonic acid, 2-(meth)acryloyl propane sulfonic acid, or 2-(meth)acrylamide-2-methylpropane sulfonic acid, and salts thereof; nonionic hydrophilic monomers such as (meth)acrylamide, N-substituted (meth)acrylate, 2-hydroxyethyl(meth)acrylate, 2-hydroxypropyl(meth)acrylate, methoxypolyethyleneglycol(meth)acrylate, or polyethyleneglycol(meth)acrylate; and amino group-containing unsaturated monomers such as (N,N)-dimethylaminoethyl(meth)acrylate or (N,N)-dimethylaminopropyl(meth)acrylamide, and quaternary compounds thereof.

The concentration of the water-soluble ethylenically unsaturated monomer may be about 20 parts by weight to 60 parts by weight, preferably, 40 parts by weight to 60 parts by weight with respect to 100 parts by weight of the composition for preparing a superabsorbent polymer, and it may be at an appropriate concentration, taking into consideration polymerization time and reaction conditions, etc. When the concentration of the monomer is too low, the yield of the superabsorbent polymer may be low and a problem may occur in economic feasibility. When the concentration is too high, a part thereof may precipitate or problems may be generated in the process, such as low pulverization efficiency during pulverization of the polymerized water-containing gel polymer, and physical properties of the superabsorbent polymer may deteriorate.

In addition, the degree of neutralization of the water-soluble ethylenically unsaturated monomer is 50% to 95%, preferably 70% to 85%. When the degree of neutralization of the monomer is low, the water absorbency of the superabsorbent polymer to be prepared may be lowered, and when the degree of neutralization is high, the monomer may be precipitated and it may be difficult to prepare the superabsorbent polymer.

As the alkali metal salt or alkali compound capable of neutralizing the water-soluble ethylenically unsaturated monomer, alkali metal salts such as sodium acrylate or alkali compounds such as caustic soda (NaOH) may be used.

As the polymerization initiator, depending on a polymerization method, a photopolymerization may employ a photopolymerization initiator, and a thermal polymerization may employ a thermal polymerization initiator.

However, even though the photopolymerization is performed, a certain amount of heat may be generated by UV irradiation or the like, and also generated with the polymerization reaction which is an exothermic reaction. Therefore, the thermal polymerization initiator may be further used.

The thermal polymerization initiator is not particularly limited, but one or more selected from the group consisting of persulfate-based initiators, azo-based initiators, hydrogen peroxide, and ascorbic acid may be preferably used. Specifically, examples of the persulfate-based initiators may include sodium persulfate ($Na_2S_2O_8$), potassium persulfate ($K_2S_2O_8$), ammonium persulfate (($NH_4)_2S_2O_8$) or the like. Examples of the azo-based initiators may include 2,2-azobis(2-amidinopropane) dihydrochloride, 2,2-azobis-(N,N-dimethylene)isobutyramidine dihydrochloride, 2-(carbamoylazo)isobutylonitrile, 2,2-azobis[2-(2-imidazolin-2-yl)propane] dihydrochloride, 4,4-azobis-(4-cyanovaleric acid) or the like.

The photopolymerization initiator is not particularly limited, but one or more selected from the group consisting of benzoin ether, dialkyl acetophenone, hydroxyl alkylketone, phenyl glyoxylate, benzyl dimethyl ketal, acyl phosphine, and α-aminoketone may be preferably used. Meanwhile, specific examples of acyl phosphine may include diphenyl(2,4,6-trimethylbenzoyl)phosphine oxide.

Meanwhile, the neutralization solution may further include a crosslinking agent. As the crosslinking agent, a crosslinking agent having one or more functional groups capable of reacting with a water-soluble substituent of the water-soluble ethylenically unsaturated monomer while having one or more ethylenically unsaturated group; or a crosslinking agent having two or more functional groups capable of reacting with the water-soluble substituent of the monomer and/or a water-soluble substituent formed by hydrolysis of the monomer may be used.

Specific examples of the crosslinking agent may include one or more selected from the group consisting of N,N'-methylenebisacrylamide, trimethylolpropane tri(meth)acrylate, ethylene glycol di(meth)acrylate, (meth)acrylate, propylene glycol di(meth)acrylate, polyethylene glycol di(meth)acrylate, polypropylene glycol(meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, pentaerythritol tetraacrylate, triallylamine, ethylene glycol diglycidyl ether, propylene glycol, glycerin, and ethylene carbonate.

The crosslinking agent is included in an amount of 0.01 part by weight or more, 0.05 parts by weight or more, 0.1 part by weight or more, or 0.3 parts by weight or more, and 2 parts by weight or less, 1.5 parts by weight or less, or 1 part by weight or less with respect to 100 parts by weight of the water-soluble ethylenically unsaturated monomer, thereby crosslinking the polymerized polymer.

The composition for preparing a superabsorbent polymer may further include additives such as a blowing agent, a thickener, a plasticizer, a preservation stabilizer, an antioxidant, etc., as needed.

For example, the composition for preparing a superabsorbent polymer may include, as the blowing agent, one or more blowing agents selected from the group consisting of sodium bicarbonate, sodium carbonate, potassium bicarbonate, potassium carbonate, calcium bicarbonate, calcium carbonate, magnesium bicarbonate, and magnesium carbonate.

The blowing agent may be added in an amount of 0.01 part by weight or more, or 0.05 parts by weight or more, or 0.08 parts by weight or more, and 0.5 parts by weight or less, 0.3 parts by weight or less, or 0.2 parts by weight or less with respect to 100 parts by weight of the monomer.

The composition for preparing a superabsorbent polymer may include a solvent. The applicable solvent may be used without limitation in its composition, as long as it is able to dissolve the above-described components. For example, the solvent used in the preparation of the clay dispersion may be used as the solvent of the composition for preparing a superabsorbent polymer.

Alternatively, the composition for preparing a superabsorbent polymer may be prepared by mixing at once the clay; the polymer dispersant; the water-soluble ethylenically unsaturated monomer; the alkali metal salt or alkali compound capable of neutralizing the water-soluble ethylenically unsaturated monomer; and the polymerization initiator in the presence of the solvent, and even in this case, the effect of improving dispersion stability of the clay by the polymer dispersant may be obtained.

The composition for preparing a superabsorbent polymer maintains excellent dispersion stability in the composition without agglomeration of the clay particles with each other or precipitation by including the clay dispersion.

Specifically, a sedimentation rate of the clay in the composition for preparing a superabsorbent polymer is 100 μm/s or less, preferably, 50 μm/s or less, or 30 μm/s or less, or 10 μm/s or less, and 1 μm/s or more, or 5 μm/s or more. A method of measuring the sedimentation rate will be described in detail in Experimental Example below.

The composition for preparing a superabsorbent polymer includes the clay, wherein the clay maintains excellent dispersion stability due to the polymer dispersant including two or more functional groups selected from the group consisting of an amine group, a carbonyl group, and a hydroxyl group, and thus the composition may be suitably used in the preparation of the superabsorbent polymer with excellent absorption performances without a concern about discoloration.

Accordingly, provided is a method of preparing a superabsorbent polymer using the composition for preparing the superabsorbent polymer.

Specifically, the method of preparing a superabsorbent polymer includes the steps of preparing a water-containing gel polymer by performing a polymerization reaction of the composition for preparing a superabsorbent polymer by heating and/or irradiating light; preparing a base polymer by drying and pulverizing the water-containing gel polymer; and forming a surface-crosslinked layer by further crosslinking the surface of the base polymer in the presence of a surface crosslinking agent.

The method further comprises the step of incorporating the obtained superabsorbent polymer, e.g. in particulate form, in an absorbent article.

The polymerization method is largely classified into a thermal polymerization and a photopolymerization according to a polymerization energy source. When the thermal polymerization is performed, it may be commonly performed in a reactor like a kneader equipped with agitating spindles. When the photopolymerization is performed, it may be performed in a reactor equipped with a movable conveyor belt. The above-described polymerization method is an example only, and the present invention is not limited to the above-described polymerization methods.

For example, the water-containing gel polymer which is obtained by performing thermal polymerization while providing hot air to the above-described reactor like a kneader equipped with the agitating spindles or heating the reactor may have a size of centimeters or millimeters when it is discharged from an outlet of the reactor, according to the type of agitating spindles equipped in the reactor. Specifically, the size of the obtained water-containing gel polymer may vary depending on a concentration of the monomer composition fed thereto, a feeding speed or the like, and the water-containing gel polymer having a weight average particle diameter of about 2 mm to about 50 mm may be generally obtained.

Further, as described above, when the photopolymerization is performed in a reactor equipped with a movable conveyor belt, the obtained water-containing gel polymer may be usually a sheet-like water-containing gel polymer having a width of the belt. In this case, the thickness of the polymer sheet may vary depending on the concentration of the monomer composition fed thereto and the feeding speed. Usually, it is preferable to supply the monomer composition such that a sheet-like polymer having a thickness of about 0.5 cm to about 5 cm may be obtained. When the monomer composition is supplied to such an extent that the thickness of the sheet-like polymer becomes too thin, it is undesirable because the production efficiency is low, and when the thickness of the sheet-like polymer is more than 5 cm, the polymerization reaction may not evenly occur over the entire thickness because of the excessive thickness.

The water-containing gel polymer obtained by the above-mentioned method may have a water content of about 40% by weight to about 80% by weight. Meanwhile, the "water content" as used herein means a weight occupied by water with respect to the total weight of the water-containing gel polymer, which may be a value obtained by subtracting the weight of the dry polymer from the weight of the water-containing gel polymer. Specifically, the water content may be defined as a value calculated by measuring the weight loss due to evaporation of moisture in the polymer during the process of drying by raising the temperature of the polymer through infrared heating. At this time, the water content is measured under the drying conditions determined as follows: the drying temperature is increased from room temperature to about 180°C and then the temperature is maintained at 180°C, and the total drying time is set to 20 minutes, including 5 minutes for the temperature rising step.

After crosslinking-polymerizing the monomer, the base polymer power may be obtained through a process of drying, pulverizing, and classifying, etc. It is appropriate to prepare and provide the base polymer powder and a superabsorbent polymer obtained therefrom each having a particle diameter of 150 $\mu$m to 850 $\mu$m through such a process of pulverizing and classifying, etc. More specifically, at least about 95% by weight or more of the base polymer powder and the superabsorbent polymer obtained therefrom may have a particle diameter of about 150 $\mu$m to about 850 $\mu$m, and the fine particles having a particle diameter of less than 150 $\mu$m may be less than about 3% by weight.

As described above, as the particle size distribution of the base polymer powder and the superabsorbent polymer is adjusted to a desirable range, the superabsorbent polymer finally prepared may exhibit the above-described physical properties and superior liquid permeability.

On the other hand, methods of performing the drying, pulverizing, and classifying will be described in more detail as follows.

First, with regard to drying the water-containing gel polymer, as needed, a coarse pulverizing step may be further performed before drying, in order to increase the efficiency of the drying step.

In this regard, a pulverizer used here is not limited by its configuration, and specifically, it may include any one selected from the group consisting of a vertical pulverizer, a turbo cutter, a turbo grinder, a rotary cutter mill, a cutter mill, a disc mill, a shred crusher, a crusher, a chopper, and a disc cutter, but is not limited to the above-described examples.

In this regard, the coarse pulverizing step may be carried out such that the particle diameter of the water-containing gel polymer becomes about 2 mm to about 10 mm.

Pulverizing into a particle diameter of less than 2 mm is not technically easy due to the high water content of the water-containing gel polymer, and an agglomeration phenomenon between the pulverized particles may occur. Meanwhile, when the polymer is pulverized into a particle diameter of greater than 10 mm, the effect of increasing the efficiency in the subsequent drying step may be insignificant.

The water-containing gel polymer coarsely pulverized as above or the water-containing gel polymer immediately after polymerization without the coarsely pulverizing step is subjected to drying. In this regard, a drying temperature of the drying step may be about 150°C to about 250°C. When the drying temperature is lower than 150°C, it is apprehended that the drying time becomes too long and the physical properties of the superabsorbent polymer finally formed may be deteriorated. When the drying temperature is higher than 250°C, it is apprehended that only the polymer surface is excessively dried, and thus fine particles may be generated during the subsequent pulverization process, and the physical properties of the superabsorbent polymer finally formed may be deteriorated. Therefore, the drying may be preferably performed at a temperature of about 150°C to about 200°C, and more preferably, at a temperature of about 160°C to about 180°C.

Meanwhile, the drying step may be carried out for about 20 minutes to about 90 minutes, in consideration of the process efficiency, but is not limited thereto.

In the drying step, any drying method may be selected and used without limitation in view of constitution, as long as it is commonly used in the process of drying the water-containing gel polymer. Specifically, the drying step may be carried out by a method such as hot air supply, infrared irradiation, microwave irradiation or ultraviolet irradiation, etc. When the drying step as above is finished, the water content of the polymer may be about 0.1% by weight to about 10% by weight.

Next, the step of pulverizing the dried polymer obtained through the drying step is performed.

The polymer powder obtained through the pulverizing step may have a particle diameter of about 150 $\mu$m to about 850 $\mu$m. Specific examples of a pulverizer that may be used to achieve the above particle diameter may include a pin mill, a hammer mill, a screw mill, a roll mill, a disc mill, or a jog mill, etc., but are not limited to the above-described examples.

**In** order to manage the physical properties of the superabsorbent polymer powder that is finally commercialized after the pulverization step, a separate process of classifying the polymer powders obtained after the pulverization according to the particle size may be carried out. Preferably, a polymer having a particle diameter of about 150 $\mu$m to about 850 $\mu$m is classified, and only the polymer powder having such a particle diameter is subjected to a surface crosslinking reaction, followed by commercialization.

Meanwhile, after performing the process of forming the above-described base polymer powder, a surface-crosslinked layer may be formed by additionally crosslinking the surface of the base polymer powder in the presence of a surface crosslinking agent, and as a result, a superabsorbent polymer may be prepared.

As the surface crosslinking agent, a compound capable of reacting with functional groups of the polymer is used, and for example, polyalcohol compounds, polyepoxy compounds, polyamine compounds, haloepoxy compounds, condensates of haloepoxy compounds, oxazoline compounds, or alkylene carbonate compounds, etc. may be used.

Specifically, examples of the polyalcohol compounds may include one or more selected from the group consisting of di-, tri-, tetra- or polyethylene glycol, 1,3-propanediol, dipropylene glycol, 2,3,4-trimethyl-1,3-pentanediol, polypropylene glycol, glycerol, polyglycerol, 2-butene-1,4-diol, 1,4-butanediol, 1,3-butanediol, 1,5-pentanediol, 1,6-hexanediol, and 1,2-cyclohexanedimethanol.

Further, as the polyepoxy compounds, ethylene glycol diglycidyl ether, polyethylene glycol diglycidyl ether, and glycidol, etc. may be used. As the polyamine compounds, one or more selected from the group consisting of ethylenediamine, diethylenetriamine, triethylenetetraamine, tetraethylenepentamine, pentaethylenehexamine, polyethyleneimine, and polyamidepolyamine may be used.

As the haloepoxy compound, epichlorohydrin, epibromohydrin and $\alpha$-methyl epichlorohydrin may be used. Meanwhile, examples of the mono-, di- or polyoxazolidinone compound may include 2-oxazolidinone, etc.

As the alkylene carbonate compound, ethylene carbonate, etc. may be used.

These compounds may be used alone or in combination thereof.

The content of the surface crosslinking agent to be added may be properly specifically selected depending on the types of the added surface crosslinking agent or the reaction conditions, but the surface crosslinking agent may be commonly used in an amount of 0.001 part by weight to 5 parts by weight, preferably 0.01 part by weight to 2 parts by weight, and more preferably 0.05 parts by weight to 3 parts by weight with respect to 100 parts by weight of the base polymer.

Further, a method of adding the surface crosslinking agent to the base polymer powder is not limited in the constitution. For example, a method of placing the surface crosslinking agent and the base polymer powder in a reactor and mixing them, a method of spraying the surface crosslinking agent onto the base polymer powder, a method of continuously supplying the base polymer powder and the surface crosslinking agent to a mixer continuously operated and mixing them, etc. may be used.

When the surface crosslinking agent is added, water and methanol may be further added after mixing. When water and methanol are added, it is advantageous in that the surface crosslinking agent may be evenly dispersed in the base polymer powder. At this time, the amount of water and methanol to be added may be preferably controlled with respect to 100 parts by weight of the base polymer powder in order to induce uniform dispersion of the surface crosslinking agent, to prevent agglomeration of the base polymer powder, and at the same time, to optimize the surface penetration depth of the surface crosslinking agent.

The surface crosslinking reaction may be performed by heating the base polymer powder, to which the surface crosslinking agent is added, at about 160°C or higher for about 20 minutes or more. **In** particular, in order to prepare a superabsorbent polymer that more appropriately satisfies the physical properties according to one embodiment, the surface crosslinking process conditions include a maximum reaction temperature of about 180°C to 200°C, and a holding time of about 20 minutes or more, or about 20 minutes or more and 1 hour or less at the maximum reaction temperature. In addition, the heating time which is taken from the temperature at the time of the initial reaction, for example, about 160°C or higher, or about 160°C to 170°C, to the maximum reaction temperature may be controlled to about 10 minutes or more, or about 10 minutes or more and about 1 hour or less. It was confirmed that the superabsorbent polymer appropriately meeting the physical properties of one embodiment may be prepared by the satisfying the above-mentioned surface crosslinking process conditions.

A means for raising the temperature for surface crosslinking reaction is not particularly limited. Heating may be performed by providing a heating medium or by directly providing a heat source. In this regard, the kind of the applicable heating medium may be steam, hot air, a hot fluid such as hot oil or the like, but the present invention is not limited thereto. The temperature of the heating medium to be provided may be properly selected in consideration of the means of the heating medium, the heating rate, and the target temperature. Meanwhile, as the heat source to be directly provided, an electric heater or a gas heater may be used, but the present invention is not limited to these examples.

The superabsorbent polymer obtained according to the above-described preparation method may exhibit very excellent properties, in which overall physical properties, such as water retention capacity and absorbency under pressure, etc., are improved at the same time, which makes the superabsorbent polymer especially suitable for its subsequent incorporation in an absorbent article.

Absorbent articles

The superabsorbent polymer of the present disclosure is incorporated in an absorbent article, such as in an absorbent core comprised by the absorbent article.

"Absorbent article" refers to devices that absorb and contain body exudates, particularly urine and other water-containing liquids, and, more specifically, refers to devices that are placed against or in proximity to the body of the wearer to absorb and contain the various exudates discharged from the body. Absorbent articles may include diapers (for babies and infants as well as for adult incontinence), pants (for babies and infants as well as for adult incontinence). As used herein, the term "exudates" includes, but is not limited to, urine, blood, vaginal discharges, sweat and fecal matter. Preferred absorbent articles of the present invention are disposable absorbent articles, more preferably disposable diapers, disposable pants and disposable absorbent inserts.

"Absorbent core" is used herein to refer to a structure intended to be disposed between a topsheet and backsheet of an absorbent article for absorbing and storing liquid received by the absorbent article.

"Disposable" is used in its ordinary sense to mean an article that is disposed or discarded after a limited number of usage events over varying lengths of time, for example, less than 10 events, less than 5 events, or less than 2 events. If the disposable absorbent article is a diaper, a pant, absorbent insert, sanitary napkin, sanitary pad or wet wipe for personal hygiene use, the disposable absorbent article is most often intended to be disposed after single use. The used and disposed absorbent article may or may not be subsequently recycled. As used herein, the terms 'absorbent article', 'pant' and 'diaper' always also refer to disposable absorbent articles, disposable pants (as well as disposable absorbent pants), and disposable diapers.

"Diaper" and "pant" refers to an absorbent article generally worn by babies, infants and incontinent persons about the lower torso so as to encircle the waist and legs of the wearer and that is specifically adapted to receive and contain urinary and fecal waste. In a pant, as used herein, the longitudinal edges of the first and second waist region are attached to each other to a pre-form waist opening and leg openings. A pant is placed in position on the wearer by inserting the wearer's legs into the leg openings and sliding the pant absorbent article into position about the wearer's lower torso. A pant may be pre-formed by any suitable technique including, but not limited to, joining together portions of the absorbent article using refastenable and/or non-refastenable bonds (e.g., seam, weld, adhesive, cohesive bond, fastener, etc.). A pant may be pre-formed anywhere along the circumference of the article (e.g., side fastened, front waist fastened). In a diaper, the waist opening and leg openings are only formed when the diaper is applied onto a wearer by (releasable) attaching the longitudinal edges of the first and second waist region to each other on both sides by a suitable fastening system.

Absorbent articles may each include a topsheet, a backsheet, an absorbent core and optionally an acquisition-distribution system. The absorbent core is placed between the backsheet and the topsheet, the optional acquisition-distribution system is typically placed between the absorbent core and the topsheet.

A superabsorbent polymer of the present disclosure may be incorporated in the absorbent core of the absorbent article. The absorbent core may or may not include other absorbent material in addition to the superabsorbent polymer, such as non-crosslinked cellulose fibers (pulp fibers). The absorbent core may include at least 60 mass%, at least 75 mass%, at least 85 mass%, at least 95 mass%, or at least 98 mass%, or 100 mass% of the superabsorbent polymer disclosed herein.

A diaper or pant may also include elasticized leg cuffs and barrier leg cuffs, which provide improved containment of liquids and other body exudates especially in the area of the leg openings. Usually each of the leg cuffs and barrier cuffs includes one or more elastic strings.

A "feminine care absorbent article" is a personal care product used by women during menstruation to absorb and retain menses, vaginal discharge, and matters from other bodily functions related to vulva. Feminine care absorbent articles include pantiliners and sanitary napkins.

Hereinafter, preferred exemplary embodiments are provided for better understanding of the present invention. However, the following exemplary embodiments are only for illustrating the present invention, and it is apparent to those skilled in the art that various changes and modifications are possible, without departing from the scope and spirit of the present invention, as disclosed in the accompanying claims.

Examples

### <Preparation of composition for preparing superabsorbent polymer>

### Example 1-1

450 g of acrylic acid, 3 g of polyethylene glycol diacrylate 400, and 0.04 g of diphenyl(2,4,6-trimethylbenzoyl) phosphine oxide were mixed, and 580 g of a 31.5% aqueous sodium hydroxide solution were introduced to prepare an acrylic acid monomer composition.

Separately, 5 g of $ZrO_2$ beads as a pulverization medium was weighed and placed in a 10 mL vial. 12 mg of polyvinylpyrrolidone (PVP, Aldrich's PVP10; average molecular weight of 10,000) as a dispersant was added thereto, and then 24 mg of clay powder (Bentonite, BYK's OPTIGEL CK, particle diameter range of 1-5 $\mu$m, average particle diameter of 3 $\mu$m) was added. 4.7 g of the acrylic acid monomer composition was added thereto, and mixed by shaking using a shaker (JEIO TECH, SK-600) at 300 rpm for 4 hours to prepare a composition for preparing a superabsorbent polymer.

### Examples 1-2 to 1-3 and Comparative Examples 1-1 to 1-4

Compositions for preparing a superabsorbent polymer of Examples 1-2 to 1-3 and Comparative Examples 1-1 to 1-4 were prepared in the same manner as in Example 1-1, respectively, except that the type of dispersant and input amount thereof were changed as in Table 1 below.

DISPERBYK-102 (BYK) used in Comparative Example 1-2 is a phosphoric acid ester-based polymer, and Sokalan HP-20 (BASF) used in Comparative Example 1-3 is a polyethylene imine-based polymer dispersant.

Table 1

| | Type of dispersant | Input amount of dispersant (mg) | Parts by weight of dispersant with respect to 100 parts by weight of clay |
|---|---|---|---|
| Example 1-1 | PVP | 12 | 50 |
| Example 1-2 | PVP | 7.2 | 30 |
| Example 1-3 | PVP | 6 | 25 |
| Comparative Example 1-1 | - | 0 | 0 |
| Comparative Example 1-2 | DISPERBYK-102 | 12 | 50 |
| Comparative Example 1-3 | Sokalan HP-20 | 12 | 50 |
| Comparative Example 1-4 | PVP | 4.8 | 20 |

**Experimental Example 1: Evaluation of physical properties of composition for preparing superabsorbent polymer**

Physical properties of the compositions for preparing a superabsorbent polymer of Examples 1-1 to 1-3 and Comparative Examples 1-1 to 1-4 were evaluated by the following methods, respectively, and the results are shown in Table 2.

**(1) Sedimentation rate**

Sedimentation rates of clay particles of each composition of Examples 2-1 to 2-3 and Comparative Examples 2-1 to 2-4 were measured using LUMiSizer Dispersion & particle size analyzer.

In detail, 1.6 mL of the composition for preparing a superabsorbent polymer was taken, put into a 10 mm PC cell, and then mounted on a LUMiSizer to measure the transmittance profile for 400 points at an interval of 10 seconds at a temperature of 25°C, 1,500 rpm (~ 280 G). In the transmittance profile thus measured, the threshold was set to 21% and the sedimentation rate was calculated.

**(2) Dispersion stability after 48 hours**

Each 4 g of the compositions for preparing a superabsorbent polymer of Examples 2-1 to 2-3 and Comparatives Example 2-1 to 2-4 was placed in a 10 ml vial, which was placed in a vial rack, and left at a temperature of 25°C for 48 hours. Next, whether or not layer separation of the supernatant occurred was visually observed. When the layer separation occurred, it was evaluated as having no dispersion stability (X), and when no layer separation occurred, it was evaluated as having dispersion stability (O).

Table 2

|  | Sedimentation rate ($\mu$m/s) | Dispersion stability after 48 hours |
|---|---|---|
| Example 1-1 | 6.55 | O |
| Example 1-2 | 6.73 | O |
| Example 1-3 | 6.42 | O |
| Comparative Example 1-1 | 370 | X |
| Comparative Example 1-2 | 106 | X |
| Comparative Example 1-3 | 6.24 | X |
| Comparative Example 1-4 | 138 | X |

Referring to Table 2, in the compositions for preparing a superabsorbent polymer of Examples 1-1 to 1-3, to which the clay dispersion including 25 parts by weight to 50 parts by weight of PVP dispersant with respect to 100 parts by weight of clay was added, sedimentation of clay did not occur well. It was confirmed that the dispersion stability was excellent even after 48 hours.

**<Preparation of Clay dispersion and Superabsorbent polymer using the same>**

**Example 2-1**

(1) Preparation of 4% by weight of clay dispersion

In a flask, 2 g of clay (Bentonite, BYK's OPTIGEL CK, particle diameter range of 1-5 $\mu$m, average particle diameter of 3 $\mu$m) and 1 g (50 parts by weight with respect to 100 parts by weight of clay) of polyvinylpyrrolidone (Aldrich's PVP10; average molecular weight of 10,000) as a polymer dispersant were placed, and water was added such that the total weight of the mixture was 50 g. This mixture was stirred with a magnetic stirrer at 500 rpm for 12 hours to prepare 4% by weight of a clay dispersion.

(2) Preparation of superabsorbent polymer

In a 3L glass container, 450 g of acrylic acid (AA), 3 g of polyethylene glycol diacrylate 400 (PEGDA 400), 0.04 g of diphenyl(2,4,6-tribenzoyl)-phosphine oxide were placed and dissolved, and then 580 g of a 31.5% aqueous sodium hydroxide solution was added to prepare an acrylic acid monomer composition.

4% by weight of the clay dispersion prepared in (1) was added to the monomer composition such that the content of clay was 0.5 parts by weight with respect to 100 parts by weight of acrylic acid, and sodium bicarbonate (SBC) which is a carbonate-based blowing agent was added in an amount of 0.1 part by weight with respect to 100 parts by weight of acrylic acid, thereby preparing a composition for preparing a superabsorbent polymer.

1000 g of the composition for preparing a superabsorbent polymer was poured into a stainless steel container with a width of 250 mm, a length of 250 mm, and a height of 30 mm, and UV polymerization was performed for 90 seconds by irradiating ultraviolet rays (irradiation dose of 10 mV/cm$^2$) to obtain a water-containing gel polymer.

Thereafter, the sheet-type water-containing gel polymer thus obtained was put into a meat chopper to obtain a water-containing gel particle powder, which was then dried in an oven, and as a result, the water content of the dry product was 1% or less. The dry particles were pulverized with a pulverizer and classified to prepare a base polymer with a size of 150 μm to 850 μm.

To 100 g of the base polymer powder, 6 g of an aqueous solution of a surface crosslinking agent containing 3 g of ethylene carbonate was sprayed, and stirred at room temperature to evenly distribute the surface crosslinking solution on the base polymer powder. Then, the base polymer powder mixed with the surface crosslinking solution was put into a surface crosslinking reactor, which was heated to 190°C for 30 minutes, and the surface crosslinking reaction was performed at the same temperature for 15 minutes.

After the surface crosslinking process, the powder was classified with a standard mesh sieve of ASTM standard to prepare a superabsorbent polymer having a particle diameter of 150 μm to 850 μm.

**Examples 2-2 to 2-3 and Comparative Examples 2-1 to 2-3**

Each clay dispersion was prepared in the same manner as in (1) of Example 2-1, except that when preparing the clay dispersion, the type and content of the clay and dispersant were adjusted as shown in Table 3 below. Then, the superabsorbent polymers of Examples 2-2 to 2-3 and Comparative Examples 2-1 to 2-3 were prepared in the same manner as (2) of Example 2-1.

Table 3

|  | Clay | Dispersant | Parts by weight of clay with respect to 100 parts by weight of AA | Parts by weight of dispersant with respect to 100 parts by weight of clay |
|---|---|---|---|---|
| Example 2-1 | Bentonite | PVP | 0.5 | 50 |
| Example 2-2 | Bentonite | PVP | 0.25 | 100 |
| Example 2-3 | Bentonite | PVP | 0.25 | 50 |
| Comparative Example 2-1 | Bentonite | - | 0.5 | 0 |
| Comparative Example 2-2 | Bentonite | Sokalan HP-20 | 0.5 | 50 |
| Comparative Example 2-3 | Bentonite | Sokalan HP-20 | 1.0 | 50 |

**Experimental Example 2: Evaluation of physical properties of superabsorbent polymer**

Physical properties of the superabsorbent polymers of Examples 2-1 to 2-3 and Comparative Examples 2-1 to 2-3 were evaluated by the following methods, respectively, and the results are shown in Table 4.

(1) Effective Absorption Capacity (EFFC)

The EFFC value was calculated according to the following equation.

$$EFFC = 1/2 \ (CRC+AUP)$$

In the equation, CRC and AUP were measured by the following methods.

a) Measurement of Centrifuge Retention Capacity (CRC)

CRC of the superabsorbent polymer was measured in accordance with EDANA method WSP 241.2.

In detail, from the polymers obtained through Examples and Comparative Examples, polymers classified with a sieve of #30-50 were obtained. This superabsorbent polymer $W_0(g)$ (about 0.2 g) was uniformly put in a non-woven bag, followed by sealing, and then immersed in physiological saline (0.9% by weight) at room temperature. After 30 minutes, water was drained from the bag using a centrifuge for 3 minutes under the condition of 250 G, and the weight $W_2(g)$ of the bag was measured. In addition, after performing the same procedure without using the polymer, the weight $W_1(g)$ at that time was measured.

CRC (g/g) was calculated using the obtained weights according to the following equation.

$$CRC\ (g/g) = \{[W_2(g) - W_1(g)]/W_0(g)\} - 1$$

b) Measurement of Absorbency Under Pressure (AUP)

AUP of 0.7 psi of the superabsorbent polymer was measured in accordance with EDANA method WSP 242.2.

First, when measuring absorbency under pressure, the classified polymer used at the time of CRC measurement was used.

In detail, a 400 mesh stainless steel net was installed in the bottom of a plastic cylinder having an internal diameter of 60 mm. The superabsorbent polymer $W_0(g)$ (0.90 g) was uniformly scattered on the stainless steel net under conditions of room temperature and humidity of 50%. A piston capable of uniformly providing a load of 0.7 psi was put thereon, in which an external diameter of the piston was slightly smaller than 60 mm, there was no gab between the internal wall of the cylinder and the piston, and the jig-jog of the cylinder was not interrupted. At this time, the weight $W_4(g)$ of the apparatus was measured.

After putting a glass filter having a diameter of 90 mm and a thickness of 5 mm in a petri dish having a diameter of 150 mm, a physiological saline solution consisting of 0.9% by weight of sodium chloride was poured until the surface level of the physiological saline solution became equal to the upper surface of the glass filter. A sheet of filter paper having a diameter of 90 mm was put on the glass filter. The measurement apparatus was mounted on the filter paper, thereby getting the liquid absorbed under the load for 1 hour. 1 hour later, the weight $W_5(g)$ was measured after lifting the measurement apparatus up.

AUP(g/g) (g/g) was calculated from the obtained weights according to the following equation.

$$AUP(g/g) = [W_5(g) - W_4(g)]/W_3(g)$$

(2) Color b

The color b value of the superabsorbent polymer was measured in accordance with ASTM D2985 standard.

Table 4

|  | EFFC | Color b |
|---|---|---|
| Example 2-1 | 27.4 | 5.0 |
| Example 2-2 | 27.4 | 5.3 |
| Example 2-3 | 27.3 | 4.9 |
| Comparative Example 2-1 | 26.8 | 4.8 |
| Comparative Example 2-2 | 27.4 | 8.6 |
| Comparative Example 2-3 | 27.0 | 11.3 |

Referring to Table 4, in Comparative Example 2-1, the dispersant was not included, and thus the dispersion stability of the clay was reduced, and as a result, the EFFC value was significantly reduced.

On the other hand, when Examples 2-1 to 2-3 and Comparative Examples 2-2 and 2-3 were compared, each using the clay dispersion including the dispersant together with clay, Comparative Examples 2-2 and 2-3 using Sokalan HP-20 as the dispersant were found to show higher color b value than Examples 2-1 to 2-3 using PVP.

From the above results, it was be confirmed that the clay dispersion of the present invention may improve the absorption properties of the prepared superabsorbent polymer due to excellent dispersion stability of the clay in the composition for preparing a superabsorbent polymer. It was also confirmed that the clay dispersion of the present invention does not cause discoloration of the polymer when used in superabsorbent polymers, and thus it is suitable for manufacturing high-quality products.

**Claims**

1. Method of making an absorbent article comprising superabsorbent polymer, wherein the superabsorbent polymer is prepared by using a composition, the composition comprising a clay dispersion; a water-soluble ethylenically unsaturated monomer; an alkali metal salt or alkali compound capable of neutralizing the water-soluble ethylenically unsaturated monomer; and a polymerization initiator;
the clay dispersion comprising:

   a solvent;
   a clay; and
   a polymer dispersant including two or more functional groups selected from the group consisting of an amine group, a carbonyl group, and a hydroxyl group,
   wherein the polymer dispersant is included **in an** amount of more than 20 parts by weight to not more than 200 parts by weight with respect to 100 parts by weight of the clay;
   wherein, in the composition, the content of the clay is from 0.1 part by weight to 1 part by weight with respect to 100 parts by weight of the ethylenically unsaturated monomer; and
   wherein, in the composition, the clay has a sedimentation rate of 100 $\mu$m/s or less, when measured in accordance with the method defined in the description.

2. The method of claim 1, wherein, in the clay dispersion, the polymer dispersant is included in an amount of from 25 parts by weight to 100 parts by weight with respect to 100 parts by weight of the clay.

3. The method of claim 1 or 2, wherein the clay is one or more selected from the group consisting of montmorillonite, saponite, nontronite, laponite, beidellite, hectorite, vermiculite, magadite, bentonite, kaolin, serpentine, and mica.

4. The method of any of the preceding claims, wherein an average particle diameter of the clay is 0.025 $\mu$m to 10 $\mu$m.

5. The method of any of the preceding claims, wherein, in the clay composition, the polymer dispersant includes an amine group and a carbonyl group.

6. The method of any of the preceding claims, wherein, in the clay composition, the polymer dispersant is one or more selected from the group consisting of polyvinylpyrrolidone, polyacrylamide, N-acetyl polyimine, and polyaminoacrylate.

7. The method of any of the preceding claims, wherein, in the composition, the water-soluble ethylenically unsaturated monomer is one or more selected from the group consisting of anionic monomers such as acrylic acid, methacrylic acid, maleic anhydride, fumaric acid, crotonic acid, itaconic acid, 2-acryloyl ethane sulfonic acid, 2-methacryloyl ethane sulfonic acid, 2-(meth)acryloyl propane sulfonic acid, or 2-(meth)acrylamide-2-methylpropane sulfonic acid, and salts thereof;
nonionic hydrophilic monomers such as (meth)acrylamide, N-substituted (meth)acrylate, 2-hydroxyethyl(meth)acrylate, 2-hydroxypropyl(meth)acrylate, methoxypolyethyleneglycol(meth)acrylate, or polyethyleneglycol(meth)acrylate; and amino group-containing unsaturated monomers such as (N,N)-dimethylaminoethyl(meth)acrylate or (N,N)-dimethylaminopropyl(meth)acrylamide, and quaternary compounds thereof.

8. The method of any of the preceding claims, wherein the method comprises the further step of incorporating the superabsorbent polymer in the absorbent article.

**Patentansprüche**

1. Verfahren zum Erzeugen eines Absorptionsartikels, umfassend Superabsorber-Polymer, wobei das Superabsorber-Polymer durch Verwenden einer Zusammensetzung hergestellt wird, die Zusammensetzung umfassend eine Tondispersion; ein wasserlösliches ethylenisch ungesättigtes Monomer; ein Alkalimetallsalz oder eine Alkaliverbindung, die in der Lage ist, das wasserlösliche ethylenisch ungesättigte Monomer zu neutralisieren; und einen Polymerisationsinitiator;

die Tondispersion umfassend:

ein Lösungsmittel;

einen Ton; und

ein Polymerdispergiermittel, das zwei oder mehr Funktionsgruppen einschließt, die ausgewählt sind aus der Gruppe, bestehend aus einer Amingruppe, einer Carbonylgruppe und einer Hydroxygruppe,

wobei das Polymerdispergiermittel in einer Menge von mehr als 20 Gewichtsteilen bis nicht mehr als 200 Gewichtsteilen bezogen auf 100 Gewichtsteile des Tons eingeschlossen ist;

wobei in der Zusammensetzung der Gehalt des Tons von 0,1 Gewichtsteil bis 1 Gewichtsteil bezogen auf 100 Gewichtsteile des ethylenisch ungesättigten Monomers beträgt; und

wobei in der Zusammensetzung der Ton eine Sedimentationsrate von 100 μm/s oder weniger aufweist, wenn gemessen gemäß dem in der Beschreibung definierten Verfahren.

2. Verfahren nach Anspruch 1, wobei in der Tondispersion das Polymerdispergiermittel in einer Menge von 25 Gewichtsteilen bis 100 Gewichtsteilen bezogen auf 100 Gewichtsteile des Tons eingeschlossen ist.

3. Verfahren nach Anspruch 1 oder 2, wobei der Ton einer oder mehrere ist, der ausgewählt ist aus der Gruppe, bestehend aus Montmorillonit, Saponit, Nontronit, Laponit, Beidellit, Hectorit, Vermiculit, Magadiit, Bentonit, Kaolin, Serpentin und Glimmer.

4. Verfahren nach einem der vorstehenden Ansprüche, wobei ein durchschnittlicher Teilchendurchmesser des Tons 0,025 μm bis 10 μm beträgt.

5. Verfahren nach einem der vorstehenden Ansprüche, wobei in der Tonzusammensetzung das Polymerdispergiermittel eine Amingruppe und eine Carbonylgruppe einschließt.

6. Verfahren nach einem der vorstehenden Ansprüche, wobei in der Tonzusammensetzung das Polymerdispergiermittel eines oder mehrere ist, das ausgewählt ist aus der Gruppe, bestehend aus Polyvinylpyrrolidon, Polyacrylamid, N-Acetylpolyimin und Polyaminoacrylat.

7. Verfahren nach einem der vorstehenden Ansprüche, wobei in der Zusammensetzung das wasserlösliche ethylenisch ungesättigte Monomer eines oder mehrere ist, das ausgewählt ist aus der Gruppe, bestehend aus anionischen Monomeren wie Acrylsäure, Methacrylsäure, Maleinsäureanhydrid, Fumarsäure, Krotonsäure, Itaconsäure, 2-Acryloylethansulfonsäure, 2-Methacryloylethansulfonsäure, 2-(Meth)acryloylpropansulfonsäure oder 2-(Meth)acrylamid-2-methylpropansulfonsäure und Salzen davon; nichtionischen hydrophilen Monomeren wie (Meth)acrylamid, N-substituiertes (Meth)acrylat, 2-Hydroxyethyl(meth)acrylat, 2-Hydroxypropyl(meth)acrylat, Methoxypolyethylenglykol(meth)acrylat oder Polyethylenglykol(meth)acrylat; und aminogruppenhaltigen ungesättigten Monomeren wie (N,N)-Dimethylaminoethyl(meth)acrylat oder (N,N)-Dimethylaminopropyl(meth)acrylamid und quartäre Verbindungen davon.

8. Verfahren nach einem der vorstehenden Ansprüche, wobei das Verfahren den weiteren Schritt eines Beimischens des Superabsorber-Polymers in den Absorptionsartikel umfasst.

**Revendications**

1. Procédé de fabrication d'un article absorbant comprenant un polymère superabsorbant, dans lequel le polymère superabsorbant est préparé à l'aide d'une composition, la composition comprenant une dispersion d'argile ; un monomère à insaturation éthylénique hydrosoluble ; un sel métallique alcalin ou un composé alcalin capable de neutraliser le monomère à insaturation éthylénique hydrosoluble ; et un initiateur de polymérisation ; la dispersion d'argile comprenant :

un solvant ;

une argile ; et

un dispersant polymère comportant deux groupes fonctionnels ou plus choisis dans le groupe constitué d'un groupe amine, d'un groupe carbonyle et d'un groupe hydroxyle,
dans lequel le dispersant polymère est inclus en une quantité de plus de 20 parties en poids à pas plus de 200 parties en poids par rapport à 100 parties en poids de l'argile ;
dans lequel, dans la composition, la teneur en argile va de 0,1 partie en poids à 1 partie en poids par rapport à 100 parties en poids du monomère à insaturation éthylénique ; et
dans lequel, dans la composition, l'argile a une vitesse de sédimentation de 100 μm/s ou moins, lorsqu'elle est mesurée conformément au procédé défini dans la description.

2. Procédé selon la revendication 1, dans lequel, dans la dispersion d'argile, le dispersant polymère est inclus en une quantité allant de 25 parties en poids à 100 parties en poids par rapport à 100 parties en poids de l'argile.

3. Procédé selon la revendication 1 ou 2, dans lequel l'argile est un ou plusieurs choisis dans le groupe constitué de montmorillonite, saponite, nontronite, laponite, béidellite, hectorite, vermiculite, magadite, bentonite, kaolin, serpentine et mica.

4. Procédé selon l'une quelconque des revendications précédentes, dans lequel un diamètre moyen de particules de l'argile va de 0,025 μm à 10 μm.

5. Procédé selon l'une quelconque des revendications précédentes, dans lequel, dans la composition d'argile, le dispersant polymère comporte un groupe amine et un groupe carbonyle.

6. Procédé selon l'une quelconque des revendications précédentes, dans lequel, dans la composition d'argile, le dispersant polymère est un ou plusieurs choisis dans le groupe constitué de polyvinylpyrrolidone, polyacrylamide, N-acétyl polyimine, et polyaminoacrylate.

7. Procédé selon l'une quelconque des revendications précédentes, dans lequel, dans la composition, le monomère hydrosoluble à insaturation éthylénique est un ou plusieurs choisis dans le groupe constitué de monomères anioniques tels que l'acide acrylique, l'acide méthacrylique, l'anhydride maléique, l'acide fumarique, l'acide crotonique, l'acide itaconique, l'acide 2-acryloyl éthane sulfonique, l'acide 2-méthacryloyl éthane sulfonique, l'acide 2-(méth)acryloyl propane sulfonique, ou l'acide 2-(méth)acrylamide-2-méthylpropane sulfonique, et des sels de ceux-ci ;
des monomères hydrophiles non ioniques tels que le (méth)acrylamide, le (méth)acrylate N-substitué, le 2-hydroxyéthyle(méth)acrylate, le 2-hydroxypropyle(méth)acrylate, le méthoxypolyéthylèneglycol(méth)acrylate, ou le polyéthylène glycol(méth)acrylate ; et des monomères insaturés contenant un groupe amino tels que le (N,N)-diméthylaminoéthyle(méth)acrylate ou le (N,N)-diméthylaminopropyle(méth)acrylamide, et des composés quaternaires de ceux-ci.

8. Procédé selon l'une quelconque des revendications précédentes, dans lequel le procédé comprend l'étape supplémentaire consistant à incorporer le polymère superabsorbant dans l'article absorbant.

FIG. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3391960 A
- WO 2009041870 A